# EUROPEAN PATENT SPECIFICATION

(11) **EP 1 748 711 B1**
(45) Date of publication and mention of the grant of the patent: **23.01.2008**
(21) Application number: 05742552.2
(22) Date of filing: 09.05.2005
(51) Int. Cl.: A47D 9/02, A61M 21/00

(54) **SLEEP INDUCING AND/OR COMFORTING DEVICE FOR INFANTS**
VORRICHTUNG, UM KLEINKINDER ZUM EINSCHLAFEN ZU BRINGEN UND/ODER ZU TRÖSTEN
DISPOSITIF PERMETTANT D'INDUIRE LE SOMMEIL CHEZ LES BEBES ET/OU DE LES RECONFORTER

(30) Priority: 11.05.2004 GB 0410537
(43) Date of publication of application: 07.02.2007
(73) Proprietor: Glatt, Julian Charles, New York 10901 (US)
(72) Inventor: GLATT, Julian, Charles, Suffern, New York 10901 (US); EDGERLEY, David, Anthony, London NW6 1HH (GB)
(74) Representative: Gilmour, David Cedric Franklyn
(86) International application number: PCT/GB2005/001782
(87) International publication number: WO 2005/107534

(56) References cited:
- DE-U1- 20 205 187
- GB-A- 2 359 994
- US-A- 4 048 684
- US-A- 4 066 072

## Description

The present invention relates to a device for inducing sleep and/or comforting infants and more particularly a device for inducing sleep and/or comforting infants by inducing a vibrational motion to the infant simulating the motion an infant experiences when travelling in a moving buggy. Parents know, through experience, that such movements can help calm restless babies. Typical buggy movements have been recorded and measured, and have been found to cover a frequency spectrum ranging from sub 1 Hz to approximately 30 Hz, with peaks at about 1 - 4Hz (a walking rhythm), and at 12 - 18 Hz (activity and motion), with lower amplitude, random gentle vibrations filling out the remainder of the spectrum as 'brown noise'.

Whilst prior art devices, such as that disclosed in GB 2,359,994, are known comprising an air inflatable bladder which can be placed beneath an infant and connected to a reciprocating pump for generating cyclic pulses to simulate the rhythm of a mother's heartbeat as experienced by a baby in the womb, such prior art devices are not suitable for reproducing the frequency range or magnitude of the complex vibrations experienced by an infant when travelling in a buggy. The prior art devices cannot produce the vibrations experienced by an infant travelling in a moving vehicle, which are also known to induce sleep in infants. Parents can use the present invention to simulate all these types of vibrations.

US 4066072 discloses a mattress for infants which supports the whole child and has a rotary fluid pump which pumps fluid into the mattress via an inlet conduit and the fluid is returned via a separate conduit. WO 0213753 discloses a pulsating device which circulates water via multiple water transfer tubes in a foam-filled bed or mattress to prevent buckling and impede water flow.

Alternative devices such as bassinets and baby chairs with built-in vibration inducing means are commercially available and marketed as having a calming and sleep inducing effect; generally the said vibration inducing means are battery powered, motor driven, eccentrically rotating bob weights, providing one or more constant speeds of rotation and frequencies of vibration. The vibrations these devices induce are repetitive, insistent and restricted to the set frequency; they do not simulate well the type of motion a baby will experience when being carried in a buggy, a moving vehicle, or by a walking person. The movements the present invention seeks to induce are gentler, more variable and made up of a complex spectrum of frequencies.

According to the present invention there is provided a device for comforting and/or inducing sleep in an infant comprising a hollow body disposable at least partially beneath an infant's body or beneath a mattress or other body upon which an infant is laid, said hollow body containing a liquid and having at least one moveable or flexible wall portion or region permitting the body to change in volume in response to a change in the pressure of the liquid within the hollow body, said hollow body being in liquid communication with a signal generator by means of an elongate tube whereby the signal generator can be placed at a location remote from the hollow body, said signal generator, in use, producing pressure pulses in the liquid contained within said hollow body of a predetermined pattern of varied frequency and magnitude, said pressure pulses causing vibrational movement of the moveable or flexible wall portion or region of the hollow body of a frequency and magnitude such as preferably simulating the motion experienced by an infant when travelling in or being carried by a buggy or other moving vehicle. Preferably at least a portion of the elongate tube is flexible. The device of the invention defines a closed liquid system which transmits the movements created by the signal generator to the hollow body disposed under the infant by means of pressure pulses and small oscillations in the liquid. The liquid carrying cavities of the device are as small as is compatible with transmitting these movements efficiently and accurately; these cavities are not filled with foam nor any other material (other than liquid) that may adversely affect the transmission of the signals in contrast to one of the prior art disclosures.

Preferably a single elongate tube is provided although it is considered possible to have multiple tubes leading to multiple bags/hollow bodies (even smaller ones), or more than one tube leading to one bag, so long as the water does not circulate.

In a preferred embodiment the signal generator comprises a closed chamber and an electrically powered voice coil or solenoid operatively connected to a flexible or moveable region of said closed chamber, said closed chamber being in fluid communication with said hollow body by means of said elongate tube, said voice coil or solenoid being operatively connected to a programmable electronic controller whereby said flexible or moveable region of said closed chamber can be reciprocated at predetermined patterns or varied frequency and magnitude. Preferably the signal generator is capable of producing vibration of the moveable or flexible region of the hollow body at a frequency of between 1 and 40 Hz (1-40 cycles per second) contoured noise. Note that the complex vibrations needed are created from the combination of a contoured spectrum of frequencies (a number of different frequencies of between 1 and 40 Hz overlaid one on top of another), not just from one frequency. This is what is known as 'noise', and is the type of vibrations the infant encounters in a buggy or being carried. A number of excitation patterns might be provided. Preferably the signal generator and all electrical components and components emitting electromagnetic radiation are placed outside the infant's cot or bassinet, some distance from the infant.

Preferably the liquid contained within the hollow body comprises an inert, safe, relatively incompressible liquid. In a preferred embodiment the liquid is water. Preferably the total volume of the liquid contained with the device is less than 200cm³, more preferably about 100 cm³.

In one embodiment, the hollow body, to be filled only with liquid, may comprise a flexible bladder. Alternatively the hollow body may comprise a first rigid portion, and said at least one moveable or flexible wall portion or region comprises a rigid wall section joined to said first rigid portion of the hollow body by means of a flexible diaphragm whereby said first rigid portion of the hollow body and the diaphragm together define a closed chamber. The diaphragm may be clamped to said first rigid portion of the hollow body by a substantially circular plate like member with a corresponding opening within which the diaphragm sits, holding and sealing the periphery of the diaphragm against the first rigid portion of the hollow body. The said moveable rigid wall section attached to the diaphragm, when positioned under a mattress, will spread the movement of the diaphragm over a larger area of the mattress.

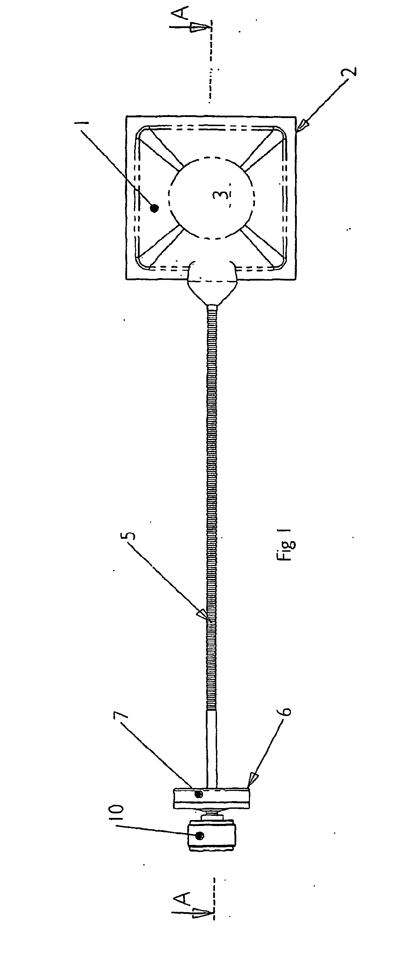

The present invention will be described further, by way of example, with reference to the accompanying drawings, in which:-
Fig. 1 is a plan view of a device according to the invention, including schematic illustration of the solenoid and electronics;
Fig. 2 is a perspective view of a device similar to that of Fig. 1 but wherein the tube is bent and leads to the signal generator which is in a housing containing the electronics, solenoid and driving chamber and other controls;

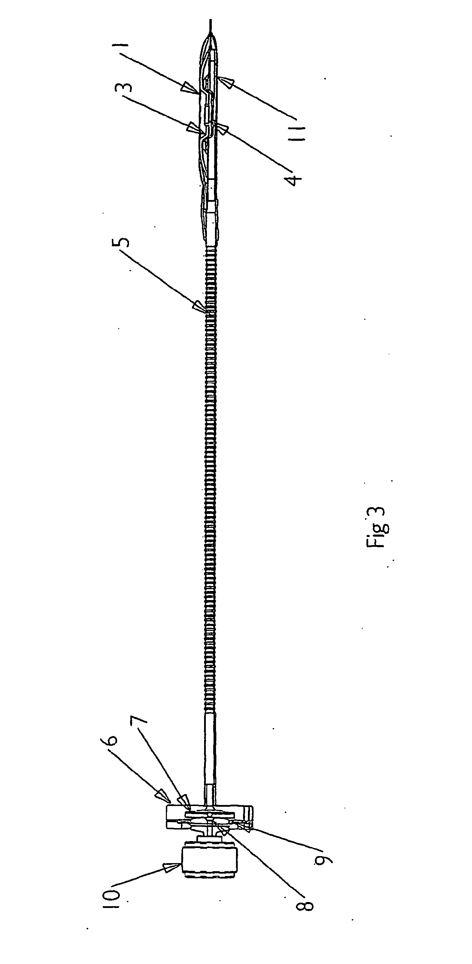

Fig. 3 is a vertical sectional view of the device of Fig. 1 taken along line A-A;
Fig. 4 is a perspective sectional view of a first part of the device of - Fig. 1 sectioned along line A-A;

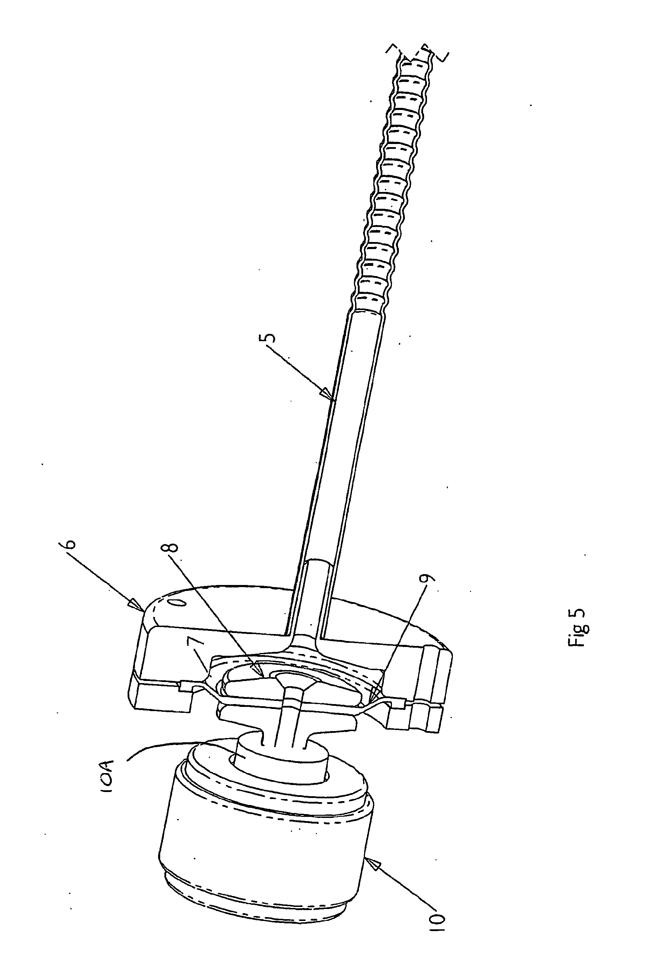

Fig. 5 is a perspective sectional view of a second part of the device of Fig. 1 sectioned along line A-A;
Fig. 6 is a perspective view similar to Fig. 4 of a slightly modified hollow body; and
Fig. 7 is an exploded view of the hollow body of Fig. 5.

As shown in the drawings, the device comprises a hollow body 1 defining a relatively thin, flat chamber or pad having a substantially square outline encased in a fabric envelope or cover 2. The hollow body 1 includes a moveable upper wall section 3 joined to the remainder of the hollow body 1 by means of a flexible diaphragm 4 such that the volume of the chamber defined by the hollow body 1 can vary in response to a variation of the pressure of a liquid within the chamber. The diaphragm 4 is of sufficient flexibility and dimensions such that the wall section 3 can reciprocate through a sufficient distance to simulate the desired vibrations. As cots and bassinets are of different sizes, and as it is intended that this device has universal application, the first part of this device does not cover the whole mattress area of a cot or bassinet. This first part of the device is likely to be placed under the cot mattress, creating vibrations through the mattress. The first part of the device should be thin and not affect the general comfort of the infant.

The hollow body 1 is connected to a first end of a single flexible, elongate tube 5 such that the interior of the tube 5 is in liquid communication with the chamber defined by the hollow body 1. This elongate tube 5, which will be laid under a mattress, must be sufficiently small in diameter not to discomfort an infant. It should also carry liquid pressure pulses efficiently so that the signal generator uses as little power as possible, as it will be driven by a low voltage transformer. As little as possible liquid in the pulsations should pass down the tube; this can be achieved by minimising the swept volume of the diaphragm of the first part of the device.

The second end of the elongate tube 5 is connected to a signal generator unit 6 (this is just the body of the device - the signal generator is not shown in detail in the drawings) comprising a driving chamber 7 in fluid communication with the interior of the tube 5, and thus the chamber defined by the hollow body 1, said driving chamber 7 having a moveable wall 8 joined to the body of the driving chamber 7 by means of a flexible diaphragm 9. The moveable wall 8 of the driving chamber forms part of or is connected to the armature 10A of a voice coil or solenoid 10 including electronic control box 10B by means of which the moveable wall 8 can be driven in response to an electrical signal supplied to the voice coil from an electronic control unit (not shown).

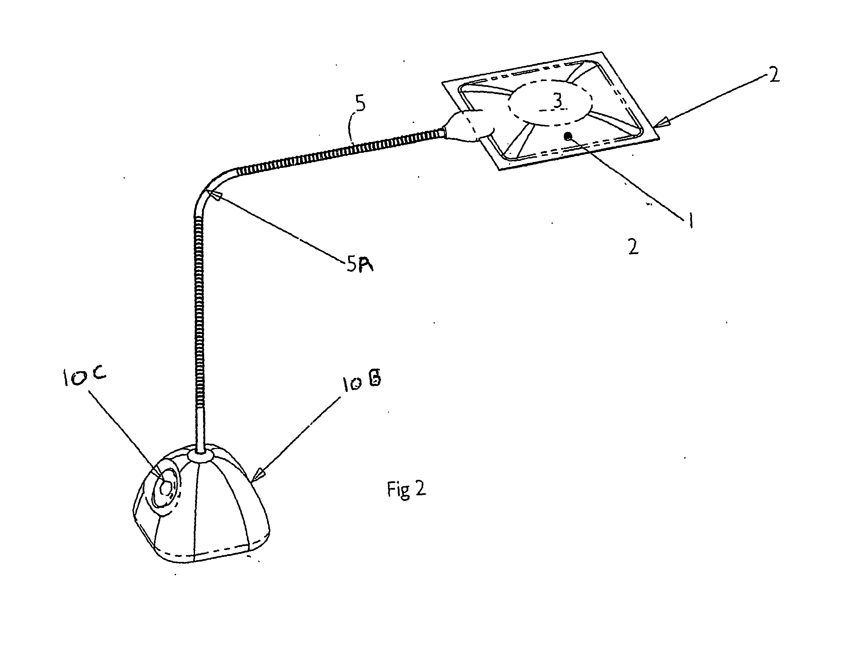

In the modification of Fig. 2, which is a perspective view illustrating casing/chamber 10B for the signal generator enclosing the electronics, solenoid and driving chamber, a control 10C is provided to alter the vibration pattern (may also include a timer to switch it off), and the tube is bent so that the signal generator enclosure can rest on the floor outside the infant's cot.

The closed volume defined by the driving chamber 7, flexible elongate tube 5 and the chamber defined by the hollow body 1 is filled with a relatively incompressible liquid such as water such that movement of the moveable wall 8 of the driving chamber 7 causes a variation in pressure in the liquid leading to a corresponding movement of the moveable wall section 3 of the hollow body 1. Thus it will be appreciated the system is a closed or sealed liquid system, without valves, and also is a non-circulatory system i.e. the liquid is not pumped through the hollow body via separate inlet and outlets. Also, the vibrations will not be simple sinusoidal ones.

Preferably the liquid comprises water such that any leaks pose no health risk to the infant. The total volume of the closed volume defined by the driving chamber 7, flexible elongate tube 5 and the chamber defined by the hollow body is approximately 100 cm³. Accordingly, even a total failure of the device leading to the loss of all water from said closed volume will only result in a minimal spillage. Using a small volume of incompressible liquid will make the transmission of vibrations from the signal generator to the first part of the device efficient. The size of the pad or hollow body will be approximately 160mm square and as thin as possible (at present about 12mm). Extendable arms (much the same shape as a ruler) or some other foldable thin structure that swing or slide out (two, three or four of them) may be attached to the top member of the hollow body, to spread the vibrations more widely under a mattress (foldable/removable for shipping).

In use, the hollow body 1 is placed beneath a mattress or other body upon which an infant is to be laid and the signal generator unit 6 is placed at a convenient location remote from said mattress by virtue of the elongate flexible tube 5. It is to be appreciated that the thin flat chamber comprising the hollow body is considerably smaller than the size of a mattress as it is intended to be used in conjunction with such, e.g. within a pocket therein, or under such to adapt such, or with another body such as a foldable fleece, or is considerably smaller than the cradle, cot/bassinet it is to be used in.

The electronic control unit is programmed with a recording of the movements and vibrations experienced by an infant when travelling in a buggy or other vehicle. The electronic control unit, which can be powered by batteries or a mains transformer, can be operated to output an electronic signal to the voice coil to generate a vibrational movement of the moveable wall 8 of the driving chamber 7. Such vibrational movement is transmitted to the moveable wall section 3 of the hollow body 1 via the liquid contained within the closed volume defined by the driving chamber 7, flexible elongate tube 5 and the chamber defined by the hollow body 1 such that the movement of the moveable wall section 3 of the hollow body 1 induces a vibrational movement to an infant resting on the mattress under which the hollow body 1 is placed, said vibrational movement having a magnitude and frequency simulating that experienced by an infant when travelling in a buggy, thus comforting the infant and/or inducing sleep in the infant.

The device can be placed beneath the normal mattress of the cot in which the baby is laid, requiring no modification of the mattress. However, it is envisaged that the hollow body 1 of the device could be placed within a pocket or recess in the mattress or be placed directly beneath the infant's body, if desired.

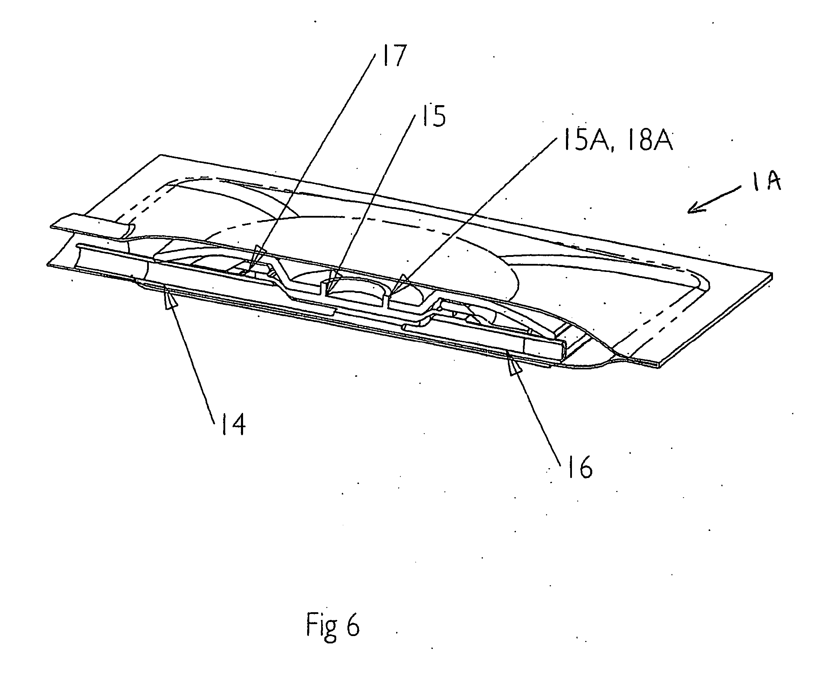

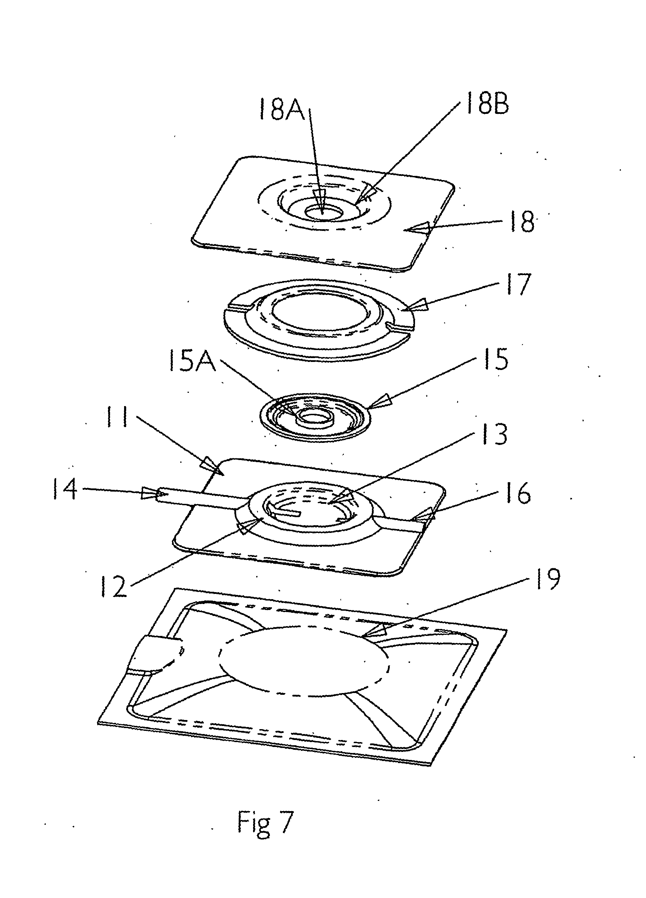

In Figs. 6 and 7, a slightly modified hollow body 1A is illustrated and comprises a rectangular or square outlined base plate 11 having a central annular raised portion 12 defining a central liquid retaining region 13 into which a tubular part 14 opens at one end and, at its other end, communicates with the first end of the single flexible elongate tube 5 (not shown in Figs. 6, 7). A flexible diaphragm 15 formed of elastomer material seats in a shoulder of portion 12 and defines the upper wall of the liquid retaining region 13. A tubular part 16 extends from the edge of base plate 11 through the portion 12 to enable filling of the device/system after which it is closed and sealed.

The diaphragm 15 is sealingly clamped in position on its seat on plate 11 by means of an annular diaphragm retainer 17 and held in position by known means (not shown). The diaphragm 15 has a central annular upstanding wall portion 15A which securely fits into a central aperture 18A in a recessed portion 18B of a top plate or moveable body 18, which is likely to be relatively stiff, which can be moved up and down (by flexing and/or other displacement) under the influence of the movement of the diaphragm 15 itself displaceable by pressure pulses in the water caused by the signal generator.

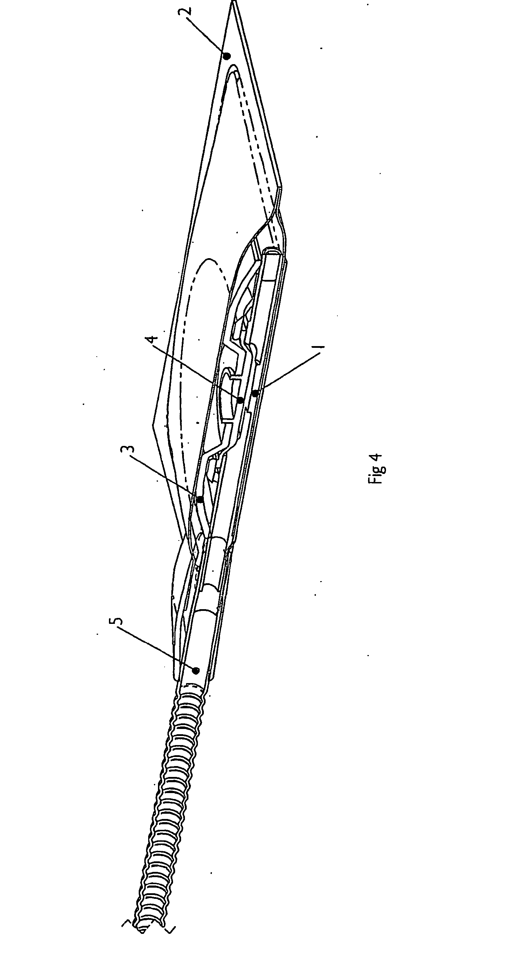

Components 11, 15, 17 and 18 are all contained within an outer fabric envelope or bag 19 of similar outline illustrated as a perspective view from above and having an opening in the left-hand side 19A. It is believed a clearer understanding will result of the construction of the hollow body 1 of Fig. 4 which is similar and shows the hollow body within its outer fabric bag.

Whilst the hollow body of the device according to the preferred embodiment is disclosed as comprising a rigid body having a moveable wall portion joined thereto by means of a flexible diaphragm, it is envisaged that the hollow body might comprise a one piece flexible bladder or similar hollow flexible body.

The signal generator drives a small diaphragm and efficiency is increased by using a single small bore tube or pipe.

The device may be operated remotely, preferably by means of a wireless controller to enable a parent to operate the device from a distance. The electronic control unit may include timer means to enable the device to be operated for a pre-determined time interval, automatically switching off after a pre-determined duration or at a predetermined time. Alternatively, or additionally, the device may be activated in response to the sound or movement of the baby and may operate for a pre-determined period thereafter.

Whilst the device according to the present invention is described for use in a cot or bassinet, it may be used in many other situations where a calming vibration is required.

Preferably the device does not create just one pattern of vibrations but rather different ones can be selected and preferably the vibrations are complex, created from a number of different frequencies of different magnitudes that are overlaid/combined to create the required vibrations (which is why a voice coil is a particularly suitable means for doing this). Further, the moving wall of the hollow body follows the pulses in the liquid accurately and the 'vibrational movement' of the moving wall of the hollow body more clearly to the patterns of excitation created by the signal generator.

## Claims

1. A device for comforting and/or inducing sleep in an infant comprising a hollow body (1) disposable at least partially beneath an infant's body or beneath or within a mattress or other body upon which an infant is laid, the space within said hollow body containing only a liquid and having at least one moveable or flexible wall portion (3) or region permitting the body to change in volume in response to a change in the pressure of the liquid within the hollow body, said hollow body being in liquid communication with a signal generator (6) by means of an elongate tube (5) whereby the signal generator can be placed at location remote from the hollow body, said signal generator, in use, producing pressure pulses in the liquid contained within said hollow body of a predetermined pattern of varied frequency and magnitude, said pressure pulses causing vibrational movement of the moveable or flexible wall portion or region of the hollow body, **characterized in that** the signal generator (6) comprises a closed chamber (7) and an electrically powered voice coil or solenoid (10) operatively connected to a flexible or moveable region (8) of said closed chamber, said closed chamber being in fluid communication with said hollow body by means of said elongate tube, said voice coil or solenoid being operatively connected to a programmable electronic controller whereby said flexible or moveable region of said closed chamber can be reciprocated at a predetermined pattern of varied frequency and magnitude.

2. A device as claimed in claim 1, wherein a single elongate tube is provided.

3. A device as claimed in any preceding claim, wherein the signal generator is capable of producing vibration of the moveable or flexible region of the hollow body at a frequency of between 1 and 40 Hz.

4. A device as claimed in any preceding claim, wherein the liquid contained within the hollow body comprises water.

5. A device as claimed in any of claims 1 to 4, wherein the flexible or moveable wall portion of the hollow body includes a flexible diaphragm forming a wall of the liquid containing part of the hollow body connected in displacing relationship to an upper moveable plate member for transmitting the vibrations to an infant or to the mattress on which an infant is placed or to be placed.

6. A device as claimed in any preceding claim, wherein the total volume of the fluid contained with the device is less than 200cm³, more preferably about 100 cm³.

7. A device as claimed in any preceding claim, wherein at least a portion of said elongate tube is flexible.

8. A device as claimed in any preceding claim, wherein the hollow body comprises a flexible bladder.

9. A device as claimed in any of claims 1 to 7, wherein said hollow body comprises a first rigid portion, said at least one moveable or flexible wall portion or region comprising a rigid wall section joined to said first rigid portion of the hollow body by means of a flexible diaphragm whereby said first rigid portion, diaphragm and rigid wall section together define a closed chamber.

10. A device as claimed in claim 9, wherein said rigid wall section includes a substantially circular liquid containing portion as part of a base plate member, said diaphragm being connected between the periphery of said circular portion and an opening in said first rigid portion.

11. A device as claimed in any of claims 1 to 10, in which the signal generator is such as to cause vibrational movement of a frequency and magnitude simulating the motion experienced by an infant when travelling in a buggy or other vehicle.

## Patentansprüche

1. Vorrichtung um Kleinkinder zu Trösten und/oder zum Einschlafen zu bringen, umfassend einen hohlen Körper (1), der zumindest teilweise unter einem Kleindkindkörper oder unter oder innerhalb einer Matratze oder eines anderen Körpers, auf welchen das Kleinkind gelegt wurde, angeordnet werden kann, wobei der Raum innerhalb des hohlen Körpers nur eine Flüssigkeit umfasst und zumindest einen bewegbaren oder flexiblen Wandteil (3) oder -bereich umfasst, welche erlauben, dass der Körper sein Volumen in Antwort auf eine Änderung des Drucks der Flüssigkeit innerhalb des hohlen Körpers ändert, der hohle Kör-per ist mittels eines langgestreckten Rohrs (5) in Flüssigkeitskommunikation mit einem Signalgenerator (6), wobei der Signalgenerator an einem Platz entfernt von dem hohlen Körper platziert werden kann, der Signalgenerator bringt, bei Verwendung, Druckpulse eines vorbestimmten Musters von verschiedenen Frequenzen und Amplituden in der Flüssigkeit, die sich innerhalb des hohlen Körpers befindet, hervor, die Druckpulse bewirken Vibrationsbewegungen des bewegbaren oder flexiblen Wandteils oder -bereichs des hohlen Körpers, da-durch **gekennzeichnet**, dass der Signalgenerator (6) eine geschlossene Kam-mer (7) und eine elektrisch betriebene Schwingspule oder gestreckte Schwing-spule (10), funktionell verbunden mit einem flexiblen oder bewegbaren Bereich (8) der geschlossenen Kammer, umfasst, die geschlossene Kammer in Fluid-kommunikation mit dem hohlen Körper mittels eines gestreckten Rohrs ist, die Schwingspule oder gestreckte Spule mit einer programmierbaren elektroni-schen Steuerung funktionell verbunden ist, wobei der flexible oder bewegbare Bereich der geschlossenen Kammer sich mit einem vorbestimmten Muster verschiedener Frequenzen und Amplituden hin und her bewegen kann

2. Eine Vorrichtung, wie beansprucht in Anspruch 1, wobei ein einziges langgestrecktes Rohr vorgesehen ist.

3. Eine Vorrichtung, wie beansprucht in einem der vorhergehenden Ansprüche, wobei der Signalgenerator geeignet ist, Vibrationen des bewegbaren oder flexiblen Bereichs des hohlen Körpers mit einer Frequenz von 1 bis 40 Hertz hervorzubringen.

4. Eine Vorrichtung, wie beansprucht in einem der vorhergehenden Ansprüche, wobei die Flüssigkeit, die in dem hohlen Körper beinhaltet ist, Wasser umfasst.

5. Eine Vorrichtung, wie beansprucht in einem der Ansprüche 1 bis 4, wobei der flexible oder bewegbare Wandteil des hohlen Körpers eine flexible Membran umfasst, die eine Wand des Flüssigkeit umfassenden Teils des hohlen Körpers ausformt, verbunden in einer verdrängenden Beziehung mit einem oberen bewegbaren Plattenmittel, um die Vibrationen an ein Kleinkind oder an eine Matratze, auf welcher das Kleinkind platziert ist oder platziert werden soll, zu übertragen.

6. Eine Vorrichtung, wie beansprucht in einem der vorhergehenden Ansprüche, wobei das Gesamtvolumen der Flüssigkeit, die in der Vorrichtung beinhaltet ist, weniger als 200 ccm und besonders bevorzugt ungefähr 100 ccm ist.

7. Eine Vorrichtung, wie beansprucht in einem der vorhergehenden Ansprüche, wobei zumindest ein Teil des langgestreckten Rohrs flexibel ist.

8. Eine Vorrichtung, wie beansprucht in einem der vorhergehenden Ansprüche, wobei der hohle Körper eine flexible Blase umfasst.

9. Eine Vorrichtung, wie beansprucht in einem der Ansprüche 1 bis 7, wobei der hohle Körper einen ersten steifen Teil umfasst, der zumindest eine bewegbare oder flexible Wandteil oder -bereich einen steifen Wandabschnitt umfasst, der mit dem ersten steifen Teil des hohlen Körpers mittels einer flexiblen Membran verbunden ist, wobei der erste steife Teil, die Membran und der steife Wandabschnitt zusammen eine geschlossene Kammer bilden.

10. Eine Vorrichtung, wie beansprucht in Anspruch 9, wobei der steife Wandabschnitt einen im Wesentlichen ringförmigen flüssigkeitsumfassenden Teil als Teil eines Basisplattenmittels beinhaltet, die Membran verbunden ist zwischen der Peripherie des ringförmigen Teils und einer Öffnung in dem ersten steifen Teil.

11. Eine Vorrichtung, wie beansprucht in einem der Ansprüche 1 bis 10, in welcher der Signalgenerator so ausgestaltet ist, dass er Vibrationsbewegungen einer Frequenz und Größe bewirkt, welche die Bewegung simulieren, die von einem Kleinkind erfahren wird, wenn es in einem Buggy oder einem anderen Fahrzeug fährt.

## Revendications

1. Dispositif pour conforter et/ou pour amener le sommeil chez un nourrisson, comprenant un corps creux (1) jetable au moins partiellement sous le corps de l'enfant ou sous ou à l'intérieur d'un matelas ou autre élément sur lequel l'enfant est couché, l'espace à l'intérieur dudit corps creux contenant uniquement un liquide et comportant au moins une portion ou une zone de paroi souple mobile (3) permettant au corps de changer de volume en réponse à une modification de la pression du liquide à l'intérieur du corps creux, ledit corps creux étant en communication, par l'intermédiaire du liquide, avec un générateur de signaux (6) au moyen d'un tube de forme allongée (5) grâce auquel le générateur de signaux peut être placé à un emplacement distant du corps creux, ledit générateur de signaux, lors de l'utilisation, produisant des impulsions de pression dans le liquide contenu à l'intérieur dudit corps creux, d'une configuration prédéterminée, de fréquence et d'amplitude variées, lesdites impulsions de pression provoquant un mouvement vibratoire de la zone ou de la portion de paroi souple du corps creux, **caractérisé par le fait que** le générateur de signaux (6) comprend une chambre fermée (7) et une bobine vocale alimenté électriquement (10), raccordée fonctionnellement avec une zone souple ou mobile (8) de ladite chambre fermée, ladite chambre fermée étant en communication par l'intermédiaire d'un fluide avec ledit corps creux au moyen dudit tube de forme allongée, ladite bobine vocale ou ledit solénoïde étant raccordés de façon fonctionnelle avec un contrôleur électrique programmable, grâce auquel ladite zone mobile ou souple de ladite chambre fermée peut être animée d'un mouvement de va-et- vient selon une configuration prédéterminée, de fréquence et d'amplitude variées.

2. Dispositif selon la revendication 1, dans lequel un seul tube de forme allongée est prévu.

3. Dispositif comme revendiqué dans l'une quelconque des revendications précédentes, dans lequel le générateur de signal est capable de produire des vibrations de la zone mobile ou souple du corps creux à une fréquence comprise entre 1 et 40 Hz.

4. Dispositif comme revendiqué dans l'une quelconque des revendications précédentes, dans lequel le liquide contenu dans le corps creux comprend de l'eau.

5. Dispositif selon l'une quelconque des revendications 1 à 4, dans lequel la portion de paroi souple ou mobile du corps creux inclut un diaphragme souple formant la paroi de la partie du corps creux contenant le liquide et raccordé de façon à pouvoir se déplacer par rapport à un élément supérieur mobile sous forme de plaque pour transmettre les vibrations à un nourrisson ou au matelas sur lequel un nourrisson est posé ou doit être posé.

6. Dispositif comme revendiqué dans l'une quelconque des revendications précédentes, dans lequel le volume total de liquide contenu dans le dispositif est inférieur à 200 cm³, de préférence environ 100 cm³.

7. Dispositif comme revendiqué dans l'une quelconque des revendications précédentes, dans lequel au moins une portion du tube de forme allongée est souple.

8. Dispositif comme revendiqué dans l'une quelconque des revendications précédentes, dans lequel le corps creux comprend une vessie souple.

9. Dispositif comme revendiqué dans l'une quelconque des revendications 1 à 7, dans lequel ledit corps creux comprend une première portion rigide, ladite au moins une portion ou zone de paroi flexible ou mobile comprenant une section de paroi rigide couplée à ladite première portion rigide du corps creux au moyen d'un diaphragme souple, lesdits première portion rigide, diaphragme et section de paroi rigide définissant ensemble une chambre fermée.

10. Dispositif comme revendiqué dans la revendication 9, dans lequel la section de paroi rigide comprend une portion substantiellement circulaire contenant le liquide comme faisant partie d'un élément de plaque de base, ledit diaphragme étant raccordé entre la périphérie de ladite portion circulaire et une ouverture se trouvant dans ladite première portion rigide.

11. Dispositif comme revendiqué dans l'une quelconque des revendications 1 à 10, dans lequel le générateur de signaux génère un mouvement vibratoire d'une fréquence et d'une amplitude simulant le mouvement ressenti par le nourrisson lorsqu'il est transporté dans une poussette ou dans un autre véhicule.
